# FASCICULE DE BREVET EUROPEEN

(11) **EP 2 961 365 B1**
(45) Date de publication et mention de la délivrance du brevet: **10.11.2021**
(21) Numéro de dépôt: 14718640.7
(22) Date de dépôt: 24.02.2014
(51) Int. Cl.: A61F 13/00, A61F 13/06, A61F 13/02

(54) **BANDE ELASTIQUE POUR BANDAGE ET PROCEDES DE FABRICATION D'UNE TELLE BANDE**
ELASTISCHE BANDAGE ZUM BANDAGIEREN UND VERFAHREN ZUR HERSTELLUNG SOLCH EINER BANDAGE
ELASTIC BANDAGE FOR BANDAGING AND METHODS FOR MANUFACTURING SUCH A BANDAGE

(30) Priorité: 27.02.2013 FR 1351738
(43) Date de publication de la demande: 06.01.2016
(73) Titulaire: THONIC INNOVATION, 43120 Monistrol-sur-Loire (FR)
(72) Inventeur: GONON, Pierre, F-43120 Monistrol-sur-Loire (FR)
(74) Mandataire: Cabinet Laurent & Charras
(86) Numéro de dépôt international: PCT/FR2014/050385
(87) Numéro de publication internationale: WO 2014/131976

(56) Documents cités:
- EP-A2- 0 217 032
- WO-A1-00/47147
- DE-U1-202012 000 529
- FR-A- 1 491 061
- FR-A1- 2 532 337
- US-A- 4 653 492
- US-A1- 2012 109 031

## Description

### DOMAINE TECHNIQUE

La présente invention concerne une bande élastique pour le bandage de parties du corps d'un patient notamment, telles que par exemple la jambe, le pied etc..., et concerne également un procédé de fabrication d'une telle bande.

### ART ANTERIEUR

Il est connu de l'état de la technique d'utiliser des bandes élastiques pour tous types de bandages de parties du corps humain, et notamment pour des techniques de soins et de protection permettant notamment de maintenir un pansement ou une attelle, ou d'assurer une contention ou une compression de la partie du corps par exemple. Le bandage peut être effectué à partir de bandes de tissu (bandage simple) ou être plus élaboré (bandage tubulaire, bandage mécanique, bandage multi-couches).

Plusieurs bandes pour bandage se trouvent sur le marché pour différents types d'applications. Il existe des bandes de contention non extensibles, en coton par exemple, des bandes élastiques à allongement court dont la valeur d'allongement est environ de 30 à 50% de sa longueur initiale, et des bandes élastiques à allongement long dont la valeur d'allongement est environ 70 à 100% de sa longueur initiale. Ces bandes élastiques constituent généralement des bandes de compression.

Ces bandes donnent entière satisfaction dans certains types d'applications, mais rencontrent parfois quelques inconvénients dans d'autres applications plus spécifiques.

Par exemple, lorsque que l'on veut traiter chez un patient certaines pathologies telles que des problèmes de lymphœdèmes, d'ulcères veineux ou des problèmes veineux en général, les bandes précitées ne sont pas réellement adaptées pour la pose par une personne non formée.

En effet, lorsque l'on réalise par exemple un bandage pour le traitement d'un lymphœdème chez un patient, il faut stopper la progression de ce lymphœdème afin d'éviter qu'il continue de grossir en volume. L'utilisation d'une bande de contention classique, à savoir inextensible, permet d'entourer le lymphœdème et de limiter son expansion par l'inextensibilité de la bande en question. Cependant, lorsque ledit lymphœdème est en voie de guérison, celui-ci diminue de volume et la bande de contention ne suit pas cette dégression, se desserre et tombe ou se détache.

En revanche, l'utilisation d'une bande élastique pour traiter ce genre de lymphœdème n'est pas non plus appropriée. En effet, lorsque le lymphœdème grossit, la bande élastique suit le mouvement et n'empêche pas le lymphœdème de grossir.

Aujourd'hui pour traiter les lymphœdèmes, des bandes à allongement court sont privilégiées, en essayant d'être le plus rigides possible pour avoir un effet de contention du lymphœdème et un effet de compression apte à suivre la dégression de celui-ci lors de la guérison. Dans la pratique, on utilise même des traitements en multicouches qui combinent les effets précités de compression et de contention. Cependant, cette accumulation de couches rend le bandage difficile à réaliser par le patient lui-même, ou toute tierce personne n'ayant pas suivi de formation préalable, et pose néanmoins un problème récurrent pour le port des vêtements, chaussures, lié à l'accumulation de couches.

Les documents FR2532337, EP0217032, DE202012000529, FR1491061 et US4653492 décrivent une bande de contention et de compression connue de l'état de la technique.

### EXPOSE DE L'INVENTION

Le problème que se propose de résoudre l'invention est donc de fournir une bande élastique capable de combiner les effets de contention et de compression précités d'une manière simple, économique, efficace, et facile à poser par le patient lui-même ou par toute tierce personne n'ayant pas suivi de formation préalable.

Un autre objectif de l'invention est de fournir une bande élastique permettant de réaliser un bandage en évitant les accumulations de couches et surépaisseurs pour ne pas gêner le port de vêtements, chaussures etc., par le patient.

Pour résoudre les problèmes précités, il a été mis au point une bande élastique pour bandage. Selon l'invention, la bande comprend des moyens permettant de limiter mécaniquement son allongement à une valeur prédéterminée. La bande élastique (1) selon l'invention est définie par la revendication 1. L'invention comprend également un procédé de fabrication de la bande élastique (1), selon la revendication 2.

De cette manière, la bande élastique comprend toutes les caractéristiques d'une bande élastique de compression et possède un allongement limité mécaniquement à une valeur prédéterminée. La force de compression d'une bande élastique dépend de son allongement. De cette manière la bande élastique selon l'invention possède une force de compression connue lorsque celle-ci est allongée jusqu'au blocage mécanique. Cela permet en outre de faciliter l'opération de bandage et de permettre notamment au patient de la réaliser lui-même. En effet, il s'agit simplement d'étirer la bande au maximum jusqu'au blocage mécanique afin d'avoir une force de compression déterminée à l'avance et de l'appliquer sur la partie du corps concernée. La bande ne sera ni trop tendue, ni trop détendue.

De plus, la bande élastique selon l'invention permet, dans le cas d'un traitement d'un lymphœdème par exemple, de stopper sa progression en volume par le blocage mécanique de la bande et également de suivre sa régression en volume par l'élasticité de la bande, afin que la bande ne prenne pas de jeu, se détache ou tombe.

Selon l'invention, les moyens permettant de limiter mécaniquement l'allongement de ladite bande sont inextensibles et rendus solidaires de la bande élastique. Les moyens sont disposés longitudinalement dans un état détendu, de sorte que l'allongement de ladite bande élastique met en tension lesdits moyens inextensibles, et limite ainsi mécaniquement l'allongement de la bande élastique à la valeur prédéterminée.

Pour homogénéiser le blocage mécanique de la bande, les moyens sont régulièrement répartis sur la surface de la bande élastique.

Les moyens se présentent sous la forme d'un fourreau en textile entourant la bande élastique.

Les moyens sont rendus solidaires de la bande élastique par l'intermédiaire d'une pluralité de coutures transversales régulièrement réparties sur la longueur de la bande élastique.

Les coutures transversales permettent notamment de définir des poches entre deux jeux de coutures transversales. Ces poches sont notamment aptes à recevoir tout principe actif ou tout élément ayant une fonction particulière, tel que de la mousse par exemple, ou bien du gel, etc...

L'invention concerne également un procédé de fabrication d'une bande élastique pour bandage.

Selon l'invention, 1e procédé est remarquable en ce qu'il comprend les étapes suivantes :
- enfilage d'un fourreau inextensible autour d'une bande élastique ;
- réalisation d'au moins un repli transversal du fourreau de manière à raccourcir d'une longueur prédéfinie sa longueur ;
- solidarisation du fourreau dans ledit état raccourci sur la bande élastique, par l'intermédiaire d'une pluralité de coutures transversales régulièrement réparties sur la longueur de la bande élastique, de sorte que l'allongement ultérieur de la bande élastique entraine la mise en tension du fourreau et limite mécaniquement l'allongement de la bande élastique à une valeur prédéterminée correspondant à la longueur prédéfinie.

### BREVE DESCRIPTION DES DESSINS

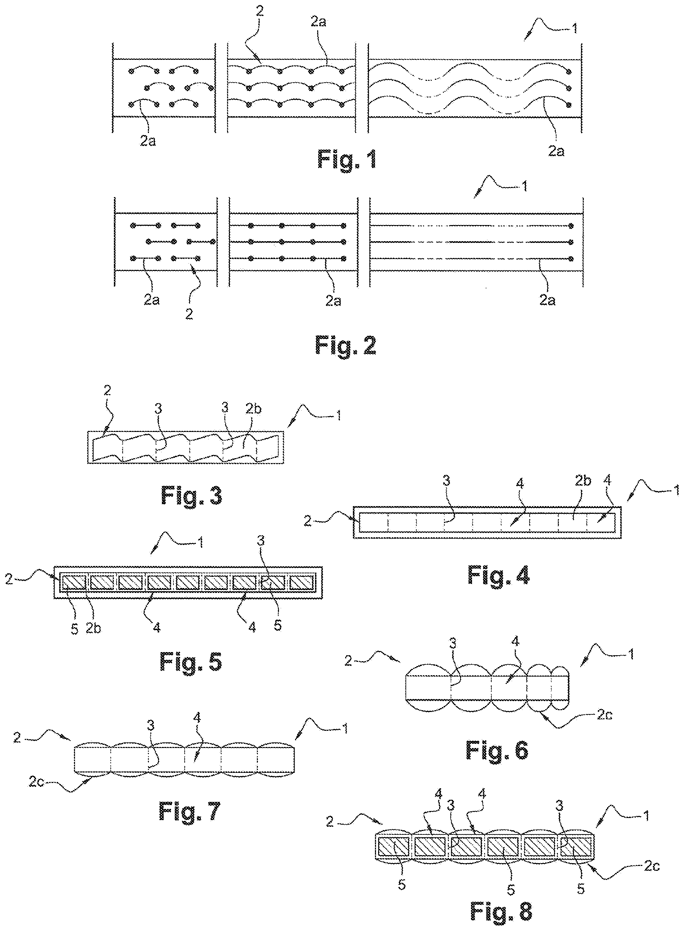

D'autres caractéristiques et avantages de l'invention ressortiront clairement de la description qui en est faite ci-après, à titre indicatif et nullement limitatif, en référence aux figures annexées, dans lesquelles les figures 6 à 12 montrent des modes de réalisation de l'invention de manière schématique :
- la figure 1 est une représentation schématique de la bande élastique selon une première forme de réalisation illustrant ladite bande élastique dans un état détendu ;
- la figure 2 est une représentation schématique similaire à celle de la figure 1, la bande élastique étant dans un état d'allongement maximum ;
- la figure 3 est une représentation schématique de la bande élastique selon une deuxième forme de réalisation illustrant ladite bande élastique dans un état détendu ;
- la figure 4 est une représentation schématique similaire à celle de la figure 3, la bande élastique étant dans un état d'allongement maximum ;
- la figure 5 est une représentation schématique similaire à celle de la figure 3, la bande élastique étant dans un état d'allongement maximum, et recevant un principe actif dans les poches formées entre deux jeux de coutures transversales ;
- la figure 6 est une représentation schématique de la bande élastique selon une troisième forme de réalisation illustrant ladite bande dans un état détendu ;
- la figure 7 est une représentation schématique similaire à celle de la figure 6, la bande élastique étant dans un état d'allongement maximum ;
- la figure 8 est une représentation schématique similaire à celle de la figure 6, la bande élastique étant dans un état d'allongement maximum, et recevant un principe actif dans les poches formées entre deux jeux de coutures transversales ;

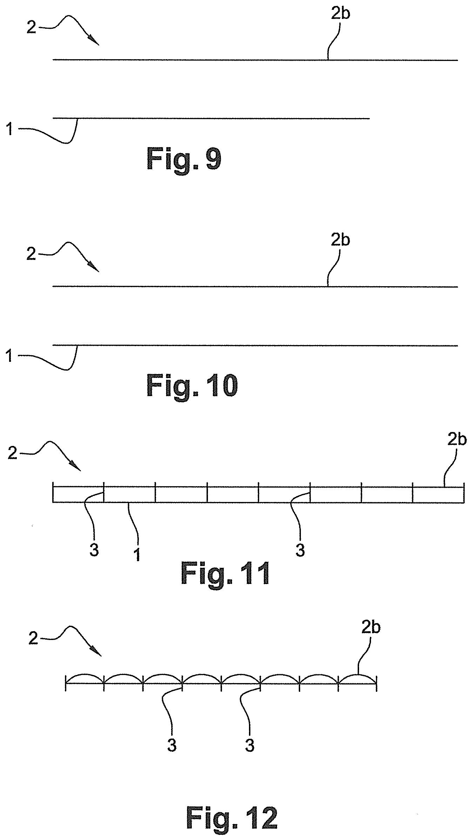

- les figures 9 à 10 sont des représentations schématiques illustrant les étapes du procédé de fabrication de la bande élastique selon l'invention, dans lesquelles la figure 9 correspond à la position initiale, c'est-à-dire dans laquelle les moyens permettant de limiter l'allongement ne sont pas solidarisés sur la bande élastiques, la figure 10 correspond à la première étape du procédé d'allongement de la bande élastique, la figure 11 correspond à la deuxième étape du procédé de solidarisation des moyens permettant de limiter l'allongement, et la figure 12 correspond à la bande élastique dans un état détendu.

### EXPOSE DETAILLE DE L'INVENTION

En référence aux figures, la bande élastique (1) selon l'invention comprend des moyens (2) permettant de limiter mécaniquement son allongement à une valeur prédéterminée. La bande élastique (1) selon l'invention est définie par la revendication 1. L'invention comprend également un procédé de fabrication de la bande élastique (1), selon la revendication 2.

La bande élastique (1) est de tout type parfaitement connu de l'homme du métier. Cette bande élastique 2. peut notamment être une bande du type bande de compression possédant une certaine force de compression en fonction de son allongement.

Les figures 1 à 5 montrent des articles qui ne font pas partie de l'invention.

En référence aux figures 1 et 2, les moyens (2) permettant de limiter mécaniquement l'allongement de la bande (1) se présentent sous la forme de fils textiles inextensibles (2a) cousus ou tricotés sur la bande élastique (1) qui est préalablement allongée à une valeur d'allongement prédéterminée, de sorte que, lorsque la bande élastique (1) est relâchée, les fils textiles (2a) se retrouvent dans un état détendu. L'allongement ultérieur de la bande (1) entraine la mise en tension des fils (2a). Cependant, les fils (2a) étant inextensibles, l'allongement de la bande (1) est limité mécaniquement. Par « état détendu » on entend que les deux extrémités d'un fil (2a) sont rapprochées l'une de l'autre. La distance qui sépare lesdites deux extrémités est inférieure à la longueur même du fil (2a). Ainsi, l'allongement possible de la bande (1) est égal à la longueur du fil (2a) moins la distance séparant les deux extrémités du fil (2a) lorsque la bande élastique (1) est relâchée. Par exemple, si la distance séparant les deux extrémités du fil (2a) équivaut à la moitié de la longueur dudit fil (2a), l'allongement possible de la bande élastique (1) sera égal à la moitié de la longueur du fil (2a).

A partir de cette forme de réalisation, plusieurs solutions peuvent être envisagées. Par exemple, une pluralité de fils textiles inextensibles (2a) et de faibles longueurs peut être cousue sur la surface de la bande élastique (1). Ces fils (2a) sont notamment disposés longitudinalement et cousus par leurs extrémités sur la bande élastique (1). Ou bien les fils (2a) peuvent être plus longs et cousus à intervalles réguliers sur la bande (1). Ou bien encore être tricotés sur celle-ci en faisant saillie d'un côté puis de l'autre de la bande (1). Les fils (2a) sont de préférence régulièrement répartis sur toute la surface de la bande (1) et peuvent être alignés ou bien disposés en quinconce ou bien de toute autre manière équivalente sans sortir du cadre de l'invention.

Selon une autre forme de réalisation, illustrée aux figures 3 à 5, les moyens (2) permettant de limiter mécaniquement l'allongement de la bande élastique (1) se présentent sous la forme d'une deuxième bande inextensible (2b) en tissu par exemple. Dans cette forme de réalisation, la bande inextensible (2b) est superposée, de préférence, sur toute la longueur de la bande élastique (1) qui est préalablement allongée à une valeur d'allongement prédéterminée. La bande inextensible (2b) est ensuite solidarisée par tous moyens appropriés sur celle-ci dans un état tendu, notamment par couture, de sorte que, lorsque la bande élastique (1) est relâchée, ladite bande inextensible (2b) se retrouve dans un état détendu. La bande inextensible (2b) est cousue sur la bande élastique (1a) par l'intermédiaire d'une pluralité de coutures transversales (3) régulièrement réparties sur la longueur de la bande inextensible (2b). L'allongement ultérieur de la bande (1) entraine la mise en tension de la bande inextensible (2b) limitant ainsi mécaniquement l'allongement de la bande élastique (1). De la même manière qu'avec les fils extensibles (2a), la longueur de l'allongement possible est égale à la longueur de la bande inextensible (2b) moins la distance séparant ses deux extrémités.

Selon l'invention illustrée aux figures 6 à 8, les moyens (2) permettant de limiter mécaniquement l'allongement de la bande élastique (1) se présentent sous la forme d'un fourreau (2c) en tissu inextensible et entourant la bande élastique (1), de préférence sur toute sa longueur. Le fourreau (2c) est notamment solidarisé par tous moyens appropriés dans un état tendu sur la bande élastique (1) qui est préalablement allongée à une valeur d'allongement prédéterminée, et notamment par couture, de sorte que, lorsque la bande élastique (1) est relâchée, le fourreau inextensible (2c) se retrouve dans un état détendu. Le fourreau inextensible (2c) est cousu sur la bande élastique (1) par l'intermédiaire d'une pluralité de coutures transversales (3), de préférence de chaque côté de la bande élastique (1), et régulièrement réparties sur la longueur du fourreau (2c). L'allongement ultérieur de la bande (1) entraine la mise en tension du fourreau inextensible (2c) limitant ainsi mécaniquement l'allongement de la bande élastique (1).

En référence aux figures 5 et 8, dans les formes de réalisation dans lesquelles les moyens (2) se présentent sous la forme d'une bande de tissu inextensible (2b) ou bien d'un fourreau en tissu (2c) conforme l'invention, ceux-ci sont rendus solidaires de la bande élastique (1) par l'intermédiaire des coutures transversales (3). Ces coutures transversales (3) sont régulièrement réparties sur la longueur de la bande inextensible (2b) ou du fourreau (2c) et définissent en outre des poches (4) entre deux coutures transversales (3). Ces poches (4) sont notamment aptes à recevoir un principe actif (5). Par principe actif (5) on entend tout produit susceptible d'avoir une fonction utile en fonction de la pathologie à traiter. Par exemple les poches (4) peuvent recevoir de la mousse afin de créer à certains endroits spécifiques des surpressions comme par exemple au niveau de la malléole lors d'un bandage de la cheville, etc... Ces poches (4) peuvent également recevoir des graines ou granules, du gel anti inflammatoire ou tout autre type de produit sans sortir du cadre de l'invention.

Lorsque les moyens (2) permettant le blocage mécanique sont des fils (2a) disposés longitudinalement, le blocage mécanique de la bande élastique (1) se fait uniquement dans la direction longitudinale d'allongement de la bande (1). En revanche, avec la bande inextensible (2b) ou le fourreau en tissu inextensible (2c), le blocage mécanique se fait dans toutes les directions d'allongement de la bande élastique (1).

La bande élastique (1) selon l'invention permet donc d'avoir toutes les caractéristiques d'une bande élastique classique avec en outre une extension contrôlée et donc une force de compression contrôlée.

La bande élastique (1) selon l'invention permet donc de pallier plusieurs inconvénients des bandes classiques de l'art antérieur. En effet, pour le traitement d'un lymphœdème par exemple, l'utilisation d'une bande élastique (1) selon l'invention permet la réalisation d'un bandage qui, d'une part, bloque la progression en volume du lymphœdème car lorsque la bande (1) atteint un allongement déterminée, elle est bloquée mécaniquement et ne peut pas s'étendre davantage, et d'autre part, lorsque le lymphœdème réduit en volume, l'élasticité de la bande (1) lui permet de suivre le lymphœdème et de ne pas prendre du jeu, se détacher ou tomber.

De plus, la bande (1) selon l'invention permet d'avoir une force de compression contrôlée car la force de compression dépend de l'allongement de la bande (1). Si on maitrise l'allongement, on maitrise ainsi la force de compression. En effet, le patient pourra réaliser le bandage lui-même, ou le faire réaliser par toute tierce personne n'ayant pas suivi de formation préalable. Il lui suffira à lui ou à cette tierce personne d'étendre la bande (1) au maximum, jusqu'au blocage mécanique et ensuite de l'appliquer sur la partie du corps concernée. Il n'y aura plus de risques que la bande (1) soit trop ou pas assez serrée. La valeur prédéterminée de l'allongement pour le blocage mécanique sera calculée en fonction de la force de compression préconisée pour l'application en question et le type de pathologie à traiter.

La fabrication de la bande (1) est également relativement simple. Plusieurs procédés peuvent être mis en oevre pour fabricer des bandes élastiques. Le procédé qui fait partie de l'invention est défini par la revendication 2.

Un des procédés de fabrication n'étant pas conforme à l'invention comprend notamment les étapes suivantes :
- allonger la bande élastique (1) à la longueur limite désirée correspondant à une valeur d'allongement prédéterminée ;
- solidariser sur la bande élastique (1) des moyens (2) inextensibles dans un état tendu.

En référence aux figures 9 à 12, on a illustré le procédé de fabrication dans le cas où les moyens (2) permettant de limiter mécaniquement l'allongement se présentent sous la forme d'une bande inextensible (2b) superposée et rendue solidaire de la bande élastique (1) par tous moyens appropriés, mais d'une manière avantageuse, par l'intermédiaire d'une pluralité de coutures transversales (3).

Ainsi, en référence à la figure 9, on est initialement en présence d'une bande élastique (1) et d'une bande inextensible (2b). Ensuite, en référence à la figure 10, la bande (1) est allongée à une valeur limite d'allongement prédéterminée correspondant à une valeur de force de compression déterminée. En référence à la figure 11, la solidarisation des moyens (2) extensibles dans un état tendu sur la bande élastique (1) allongée à la valeur d'allongement désirée permet de limiter mécaniquement et ultérieurement l'allongement de la bande (1) à ladite valeur prédéterminée et désirée.

A la fin du procédé, la bande élastique (1) est soit relâchée en référence à la figure 12, soit de préférence enroulée directement dans un état d'allongement maximum et stockée en rouleau (non représenté) de sorte que le patient ait uniquement et simplement à déposer et dérouler la bande (1) sur la partie de corps concernée, sans nécessiter de l'étirer lui-même. Ceci est un avantage notamment pour les personnes souffrant d'arthrose par exemple ou ayant des difficultés à étirer une bande élastique (1).

Une bande élastique non conforme à l'invention, peut également être fabriquée selon un autre procédé de fabrication.

Ce procédé non conforme à l'invention comprend les étapes suivantes :
- superposition d'une bande élastique (1) avec une bande inextensible (2b) ;
- réalisation d'au moins un repli transversal de la bande inextensible (2b) de manière à raccourcir d'une longueur prédéfinie la longueur de ladite bande inextensible ;
- solidarisation de la bande inextensible (2b) dans ledit état raccourci sur la bande élastique (1) de sorte que l'allongement ultérieur de la bande élastique (1) entraine la mise en tension de la bande inextensible (2b) et limite mécaniquement l'allongement de la bande élastique (1) à une valeur prédéterminée correspondant à la longueur prédéfinie.

De cette manière, la bande inextensible (2b) est solidarisée dans un état détendu sur la bande élastique (1), de sorte que l'allongement de la bande élastique (1) entraine la mise en tension de la bande inextensible (2b) et limite l'allongement de ladite bande élastique (1). La valeur prédéterminée de l'allongement correspond en d'autres termes à la différence de longueur de la bande inextensible (2b) entre son état détendu et son état tendu.

Selon l'invention, la bande élastique (1) est fabriquée selon un procédé de fabrication comprenant les étapes suivantes :
- enfilage d'un fourreau inextensible (2c) autour d'une bande élastique (1) ;
- réalisation d'au moins un repli transversal du fourreau (2c) de manière à raccourcir d'une longueur prédéfinie sa longueur ;
- solidarisation du fourreau (2c) dans ledit état raccourci sur la bande élastique (1), par l'intermédiaire d'une pluralité de coutures transversales (3) régulièrement réparties sur la longueur de la bande élastique (1), de sorte que l'allongement ultérieur de la bande élastique (1) entraine la mise en tension du fourreau (2c) et limite mécaniquement l'allongement de la bande élastique (1) à une valeur prédéterminée correspondant à la longueur prédéfinie.

Dans ce cas et de la même manière, la valeur prédéterminée de l'allongement correspond en d'autres termes à la différence de longueur du fourreau (2c) entre son état détendu et son état tendu.

Comme il ressort de ce qui précède, l'invention fournit une bande élastique (1) donnant entière satisfaction, de fabrication et de conception simple, sûre et rationnelle. La bande élastique (1) selon l'invention permet notamment au patient de réaliser son bandage lui-même, ou de le faire réaliser par toute tierce personne n'ayant pas suivi de formation préalable, en garantissant une force de compression préconisée et uniforme. La bande élastique (1) permet également de réaliser un bandage en évitant les accumulations de couches et surépaisseurs pour ne pas gêner le port de vêtements, chaussures etc. par le patient, par rapport aux bandes de l'état de la technique de conception souvent trop complexes.

## Revendications

1. Bande de contention et de compression pour bandage apte à traiter un lymphoedème ou un problème veineux ***caractérisée en ce qu**'*elle comprend une bande élastique (1) et un fourreau (2c) en textile inextensible entourant ladite bande élastique (1), et rendu solidaire de celle-ci, par l'intermédiaire d'une pluralité de coutures transversales (3) régulièrement réparties sur la longueur de la bande élastique (1), ledit fourreau étant disposé longitudinalement dans un état détendu, de sorte que l'allongement de ladite bande élastique (1) met en tension ledit fourreau inextensible (2c), et limite ainsi mécaniquement l'allongement de la bande élastique (1) à une valeur prédéterminée.

2. Procédé de fabrication d'une bande de contention et de compression selon la revendication 1,
***caractérisé en ce qu'***il comprend les étapes suivantes :
- enfilage d'un fourreau inextensible (2c) autour d'une bande élastique (1) ;
- réalisation d'au moins un repli transversal du fourreau (2c) de manière à raccourcir d'une longueur prédéfinie sa longueur ;
- solidarisation du fourreau (2c) dans ledit état raccourci sur la bande élastique (1), par l'intermédiaire d'une pluralité de coutures transversales (3) régulièrement réparties sur la longueur de la bande élastique (1), de sorte que l'allongement ultérieur de la bande élastique (1) entraine la mise en tension du fourreau (2c) et limite mécaniquement l'allongement de la bande élastique (1) à une valeur prédéterminée correspondant à la longueur prédéfinie.

## Patentansprüche

1. Ruhigstellungs- und Kompressionsverband für Bandagen zur Behandlung eines Lymphödems oder eines Venenproblems, ***dadurch gekennzeichnet, dass*** er eine elastische Binde (1) und eine Hülle (2c) aus nicht dehnbarem Stoff enthält, die diese elastische Binde (1) umgibt und mit dieser über eine Vielzahl von Quernähten (3) verbunden ist, die regelmäßig über die Länge der elastischen Binde (1) verteilt sind, die Hülle ist dabei in Längsrichtung in entspanntem Zustand angeordnet, so dass bei der Dehnung der elastischen Binde (1) diese nicht dehnbare Hülle (2c) gespannt wird und auf diese Art und Weise die Dehnung der elastischen Binde (1) mechanisch auf einen vorher festgelegten Wert begrenzt wird.

2. Verfahren zur Herstellung eines Ruhigstellungs- und Kompressionsverbandes nach Anspruch 1, ***dadurch gekennzeichnet, dass*** es die folgenden Schritte umfasst:
- Einfädeln einer nicht dehnbaren Hülle (2c) um eine elastische Binde herum (1);
- Ausführung mindestens einer Faltung in Querrichtung der Hülle (2c), um so ihre Länge um eine vorher definierte Länge zu verkürzen;
- feste Verbindung der Hülle (2c) in diesem verkürzten Zustand mit der elastischen Binde (1), über eine Vielzahl von Quernähten (3) die regelmäßig über die Länge der elastischen Binde (1) verteilt sind, so dass die spätere Dehnung der elastischen Binde (1) dazu führt, dass die Hülle (2c) gespannt wird und die Dehnung der elastischen Binde (1) mechanisch auf einen vorher festgelegten Wert begrenzt wird, der der vorher festgelegten Länge entspricht.

## Claims

1. Constrictive and compressive band for bandaging, capable of treating a lymphoedema or a vein problem, **characterised in that** it comprises an elastic band (1) and a sheath (2c) made of unstretchable textile surrounding said elastic band (1), and made integral with it, by way of a plurality of transverse seams (3) regularly distributed over the length of the elastic band (1), said sheath being disposed longitudinally in an expanded state, such that the extension of said elastic band (1) tensions said unstretchable sheath (2c), and thus mechanically limits the extension of the elastic band (1) to a predetermined value.

2. Method for manufacturing a constrictive and compressive band according to claim 1, **characterised in that** it comprises the following steps:
- threading of an unstretchable sheath (2c) around an elastic band (1);
- making at least one transverse fold in the sheath (2c) so as to shorten its length from a predefined length;
- securing the sheath (2c) in said shortened state on the elastic band (1), by way of a plurality of transverse seams (3) regularly distributed over the length of the elastic band (1), such that the subsequent extension of the elastic band (1) leads to the tensioning of the sheath (2c) and mechanically limits the extension of the elastic band (1) to a predetermined value corresponding to the predefined length.
